(19) Europäisches Patentamt
European Patent Office
Office européen des brevets

(11) Veröffentlichungsnummer: **0 000 017**
**B1**

(12) **EUROPÄISCHE PATENTSCHRIFT**

(45) Veröffentlichungstag der Patentschrift: **30.09.81**

(51) Int. Cl.³: **C 07 D 249/08,**
A 01 N 43/64

(21) Anmeldenummer: **78100029.4**

(22) Anmeldetag: **01.06.78**

(54) **1-(2-Phenyläthyl)-triazolium-Salze, Verfahren zur ihrer Herstellung sowie ihre Verwendung als Fungizide.**

(30) Priorität: **04.06.77 DE 2725314**

(43) Veröffentlichungstag der Anmeldung:
**20.12.78 Patentblatt 78/01**

(45) Bekanntmachung des Hinweises auf die Patenterteilung:
**30.09.81 Patentblatt 81/39**

(84) Benannte Vertragsstaaten:
**BE CH DE FR GB NL SE**

(56) Entgegenhaltungen:
**FR - A - 2 323 689**

(73) Patentinhaber: **BAYER AG**
**Zentralbereich Patente, Marken und Lizenzen**
**D-5090 Leverkusen 1,**
**Bayerwerk (DE)**

(72) Erfinder: **Timmler, Helmut, Dr.**
**Tersteegenweg 16**
**D-5600 Wuppertal 11 (DE)**
Erfinder: **Krämer, Wolfgang, Dr.**
**Am Eckbusch 39/162**
**D-5600 Wuppertal 1 (DE)**
Erfinder: **Büchel, Karl Heinz, Prof. Dr.**
**Bergerheide 62**
**D-5600 Wuppertal (DE)**
Erfinder: **Brandes, Wilhelm, Dr.**
**Eichendorffstrasse 3**
**D-5653 Leichlingen (DE)**
Erfinder: **Frohberger, Paul-Ernst, Dr.**
**Willi-Baumeister-Strasse 5**
**D-5090 Leverkusen (DE)**

**EP 0 000 017 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 1-(2-Phenyläthyl)-triazolium-Salze, Verfahren zu ihrer Herstellung sowie ihre Verwendung als Fungizide

Die Erfindung betrifft 1-(2-Phenyläthyl)-triazolium-Salze, ein Verfahren zu ihrer Herstellung sowie ihre Verwendung als Fungizide.

Es ist bereits bekannt geworden, daß 1-[β-Aryl-β-(R-oxy)-äthyl]-imidazole, wie z.B. 1-[β-Butoxy-β-(4'-chlorphenyl)-äthyl]-imidazol und 1-[β-Aryl-β-(R-oxy)-äthyl]-triazole, wie z.B. 1-[β-Allyloxy-β-(4'-chlorphenyl)-äthyl]-1,2,4-triazol, eine gute fungizide Wirksamkeit aufweisen (vgl. DE—OS 2 063 857 und 2 640 823). Deren Wirkung ist jedoch in bestimmten Indikationsbereichen, insbesondere bei niedrigen Aufwandmengen und -konzentrationen, nicht immer voll befriedigend. Weiterhin ist allgemein seit längerer Zeit bekannt, daß Zink-äthylen-1,2-bisdithiocarbamidat ein gutes Mittel zur Bekämpfung von pilzlichen Pflanzenkrankheiten ist [vgl. Phytopathology *33*, 1113 (1963)]. Jedoch ist dessen Einsatz als Saatgutbeizmittel nur beschränkt möglich, da es bei niedrigen Aufwandmengen und -konzentrationen wenig wirksam ist.

Es wurden nun die 1-(2-Phenyläthyl)-triazolium-Salze der allgemeinen Formel I

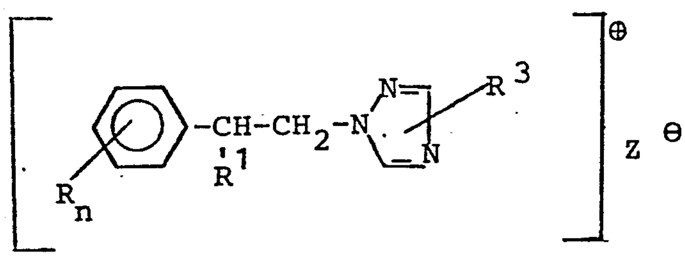

(I)

in welcher

R für Halogen, Alkyl mit 1 bis 4 Kohlenstoffatomen, gegebenenfalls durch Halogen substituiertes Phenyl oder Phenoxy steht,

n für ganze Zahlen von 0 bis 3 steht,

$R^1$ für die Gruppe —O—$R^2$, für Alkylmercapto oder Alkylcarbonyloxy mit jeweils 1 bis 4 Kohlenstoffatomen im Alkylteil steht,

$R^2$ für Alkenyl mit 2 bis 4 Kohlenstoffatomen und für gegebenenfalls durch Halogen substituiertes Benzyl steht,

$R^3$ für Alkyl mit 1 bis 6 Kohlenstoffatomen, für Pinakolyl und für gegebenenfalls durch Halogen substituiertes Benzyl oder Benzoylmethyl steht, und

Z für Halogen steht,

gefunden. Sie weisen starke fungizide Eigenschaften auf.

Weiterhin wurde gefunden, daß man die 1-(2-Phenyläthyl)-triazolium-Salze der allgemeinen Formel I erhält, wenn man 1-Phenyl-2-triazolyl-äthyl-Derivate der allgemeinen Formel II

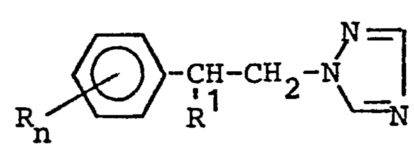

(II)

in welcher

R, $R^1$ und n die oben angegebene Bedeutung haben, mit Halogeniden der allgemeinen Formel III

$$R^3—X \qquad (III)$$

in welcher

$R^3$ die oben angegebene Bedeutung hat und

X für Halogen steht,

in Gegenwart eines Verdünnungsmittels in an sich bekannter Weise umsetzt und gegebenenfalls das Halogenid in den so erhaltenen Triazolium-Halogeniden ebenfalls in an sich bekannter Weise gegen ein anderes Halogenid austauscht.

In manchen Fällen erweist es sich als vorteilhaft, anstelle der Halogenide der allgemeinen Formel III entsprechende reaktive Ester einzusetzen, die durch Umsetzung der entsprechenden Alkohole mit den entsprechenden Säuren erhalten werden.

Ueberraschenderweise zeigen die erfindungsgemäßen 1-(2-Phenyläthyl)-triazolium-Salze eine erheblich höhere fungizide Wirksamkeit, insbesondere gegen Rost- und Mehltauarten, als die aus dem Stand der Technik bekannten 1-[β-Aryl-β-(R-oxy)-äthyl]-imidazole und -triazole, beispielsweise 1-[β-Butoxy-β-(4′-chlor-phenyl)-äthyl]-imidazol, welche chemisch und wirkungsmäßig naheliegende Stoffe darstellen, und als das Zink-äthylen-1,2-bis-dithiocarbamidat, welches ein bekannter Stoff gleicher Wirkungsrichtung ist. Die erfindungsgemäßen Wirkstoffe stellen somit eine Bereicherung der Technik dar.

Verwendet man [1-(2,4-Dichlorphenyl)-2-(1,2,4-triazol-1-yl)-äthyl]-(2,4-dichlorbenzyl)-äther und Monochlorpinakolin als Ausgangsstoffe, so kann der Reaktionsablauf durch das folgende Formelschema wiedergegeben werden:

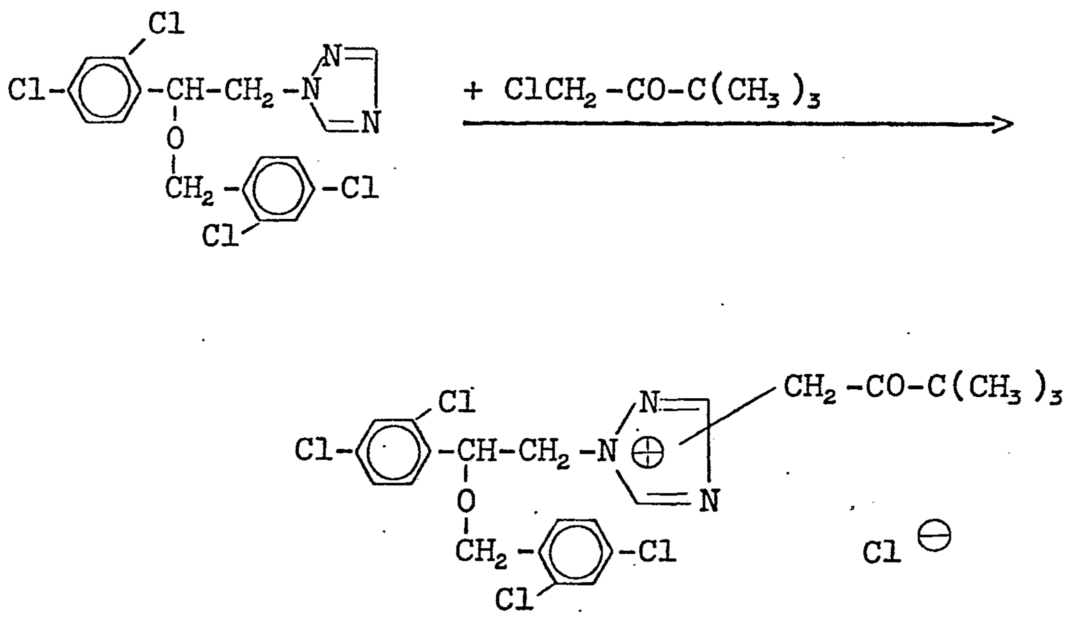

Dabei tritt der Alkylteil bevorzugt an das Stickstoffatom 4 des 1,2,4-Triazol-1-yl-Restes, wie aus den Kernresonanzdaten hervorgeht; in Abhängigkeit von der Reaktivität des Alkylhalogenids und des Ausgangsprodukts der allgemeinen Formel II kann jedoch auch eine Alkylierung am Stickstoffatom 1 des 1,2,4-Triazol-1-yl-Restes erfolgen. Beide Verbindungstypen, sowie die bei dieser Alkylierung erhaltenen Gemische werden erfindungsgemäß beansprucht.

Die als Ausgangsstoffe zu verwendenden 1-Phenyl-2-triazolyläthyl-Derivate sind durch die allgemeine Formel II allgemein definiert.

Die Ausgangsstoffe der allgemeinen Formel II sind bekannt (vgl. DE—OS 25 47 953, DE—OS 26 40 823, DE—OS 26 28 419 und DE—OS 26 45 496). Sie können nach den dort beschriebenen Verfahren erhalten werden, indem man z.B.

a) Alkanolate von 1-Hydroxy-1-phenyl-2-triazolyl-äthan-Derivaten der allgemeinen Formel IV

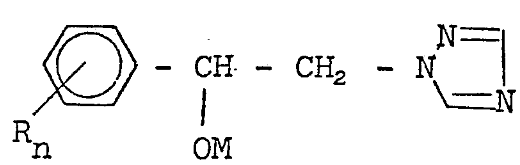

(IV)

in welcher

R und n die oben angegebene Bedeutung haben und

M für ein Alkalimetall, vorzugsweise Lithium, Natrium und Kalium, eine quarternäre Ammonium- oder Phosphoniumgruppe steht,

mit einem Halogenid der allgemeinen Formel V

$$Hal—R^2 \quad (V)$$

in welcher

$R^2$ die oben angegebene Bedeutung hat und

Hal für Chlor oder Brom steht,

in Gegenwart eines organischen Lösungsmittels, wie beispielsweise Dioxan oder Chloroform, bei Temperaturen zwischen 20 und 120°C umsetzt. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einem 1-Hydroxy-1-phenyl-2-triazolyl-äthan-Derivat ausgeht, letzteres in einem geeigneten inerten Lösungsmittel mittels Alkalimetallhydrid oder Alkalimetallamid in das Alkalimetall-alkoholat der allgemeinen Formel IV überführt, und letzteres ohne Isolierung sofort mit einem Halogenid der allgemeinen Formel V umsetzt, wobei unter Austritt von Alkalihalogenid die Verbindungen der allgemeinen Formel II in einem Arbeitsgang erhalten werden.

Nach einer weiteren bevorzugten Ausführungsform werden zweckmäßigerweise die Herstelltung der Alkanolate der allgemeinen Formel IV sowie die Umsetzung mit dem Halogenid der allgemeinen Formel V in einem Zweiphasensystem, wie beispielsweise wässrige Natron- oder Kalilauge/Toluol oder Methylenchlorid, unter Zusatz eines Phasen-Transfer-Katalysators, wie beispielsweise Ammonium- oder Phosphoniumverbindungen, durchgeführt.

Einzelne Ausgangsstoffe der allgemeinen Formel II können auch erhalten werden, wenn man

b) die den Alkanolaten der allgemeinen Formel IV zugrunde liegenden 1-Hydroxy-1-phenyl-2-triazolyl-äthane mit entsprechenden Säureanhydriden nach bekannten Methoden, z.B. in molaren Mengen in Gegenwart eines inerten organischen Lösungsmittels, beispielsweise Aceton, oder mit einem Überschuß and Säureanhydrid und in Gegenwart eines sauren oder basischen Katalysators, beispielsweise Natriumacetat, bei Temperaturen zwischen 0 und 150°C umsetzt und die Verbindungen der allgemeinen Formel II in üblicher Weise isoliert, oder

c) 1-Halogen-1-phenyl-2-triazolyl-äthane der allgemeinen Formel VI

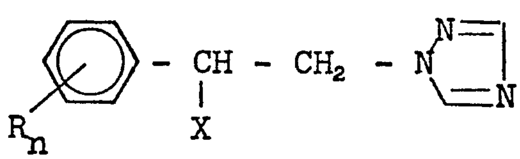

(VI)

in welcher

R und n die oben angegebene Bedeutung haben und

X für Halogen steht,

mit Alkylmercaptanen mit Alkylresten enthaltend 1 bis 4 Kohlenstoffatome in bekannter Weise in Gegenwart eines inerten organischen Lösungsmittels, beispielsweise Aceton, und in Gegenwart eines Säurebindemittels, beispielsweise Kaliumcarbonat, bei Temperaturen zwischen 60 und 100°C umsetzt.

Die den Alkanolaten der allgemeinen Formel IV zugrunde liegenden 1-Hydroxy-1-phenyl-2-triazolyl-äthan-Derivate sind bekannt (vgl. DE—OS 24 31 407 und DE—OS 25 47 953).

Die 1-Halogen-1-phenyl-2-triazolyl-äthan-Derivate der allgemeinen Formel VI sind ebenfalls bekannt (vgl. DE—OS 25 47 954).

Die außerdem als Ausgangsstoffe zu verwendenden Halogenide sind durch die allgemeine Formel III allgemein definiert.

Die Ausgangsstoffe der allgemeinen Formel III sind allgemein bekannte Verbindungen der organischen Chemie, bzw. können sie auf allgemein bekannte und übliche Weise erhalten werden.

Für die erfindungsgemäße Umsetzung kommen als Verdünnungsmittel vorzugsweise polare organische Lösungsmittel infrage. Hierzu gehören vorzugsweise Nitrile, wie Acetonitril; Sulfoxide, wie Dimethylsulfoxid; Formamide, wie Dimethylformamid; Ketone, wie Acetone; Äther, wie Diäthyläther und Tetrahydrofuran sowie Chlorkohlenwasserstoffe, wie Methylenchlorid und Chloroform.

Die Reaktionstemperaturen können beim erfindungsgemäßen Verfahren in einem größeren Bereich variiert werden. Im allgemeinen arbeitet man zwischen etwa 0 bis 120°C, vorzugsweise bei 20 bis 90°C, bzw. bei der Siedetemperatur des jeweiligen Lösungsmittels.

Bei der Durchführung des erfindungsgemäßen Verfahrens arbeitet man vorzugsweise in molaren Mengen. Die Isolierung der Verbindungen der allgemeinen Formel I erfolgt in üblicher Weise.

Der, entsprechend dem erfindungsgemäßen Verfahren, gegebenenfalls durchzuführende Halogenionen-Austausch erfolgt in allgemein bekannter Weise (vgl. DE—OS 2 504 114), indem man die 1-(2-Phenyläthyl)-triazolium-halogenide der allgemeinen Formel I z.B. mittels einer Base oder einem Anionenaustauschharz in die entsprechenden Triazoliumhydroxide überführt und diese anschließend mit einer entsprechenden Säure umsetzt.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Venturia-Arten, wie gegen den Erreger des Apfelschorfs (Fusicladium dendriticum), von echten Mehltaupilzen, beispielsweise zur Bekämpfung von Apfelmehltau (Podosphaera leucotricha) und Getreidemehltau sowie gegen andere Getreidekrankheiten verwendet werden.

Besonders hervorzuheben ist die teilweise systemische Wirkung der Stoffe. So gelingt es, Pflanzen gegen Pilzbefall zu schützen, wenn man den Wirkstoff über den Boden und die Wurzel den oberirdischen Teilen der Pflanze zuführt.

Als Pflanzenschutzmittel können die erfindungsgemäßen Wirkstoffe zur Saatgut- oder Bodenbehandlung und zur Behandlung oberirdischer Pflanzenteile benutzt werden.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyäthylen-Fettsäure-Ester, Polyoxyäthylen-Fettalkohol-Äther, z.B. Alkylaryl-polyglycol-Äther, Alkylsulfonate, Alkylsulfate, Arylsulfonate sowie Eiweißhydrolysate; als Dispergiermittel: z.B. Lignin, Sulfitablaugen und Methylcellulose.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Formulierungen enthalten im allgemeinen zwischen 0,1 und 95 Gewichtsprozent Wirkstoff, vorzugsweise zwischen 0,5 und 90%.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 100 g je cbm Boden, wie insbesondere 10 bis 200 g, erforderlich.

Die vielseitigen Verwendungsmöglichkeiten gehen aus den nachfolgenden Beispielen hervor.

Beispiel A

Podosphaera-Test (Apfel)/Protektiv

| | | |
|---|---|---|
| Lösungsmittel: | 4,7 | Gewichtsteile Aceton |
| Emulgator: | 0,3 | Gewichtsteile Alkyl-arylpolyglykoläther |
| Wasser: | 95,0 | Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Berfall, 100% bedeutet, daß die Pflanzen vollständig befallen sind.

Wirkstoffe, Wirkstoffkonzentrationen und Ergebnisse gehen aus der nachfolgenden Tabelle hervor:

Tabelle A

Podosphaera-Test (Apfel)/Protektiv

| Wirkstoff | Befall in % bei einer Wirkstoffkonzentration von |
|---|---|
| | 0,0025% |
| 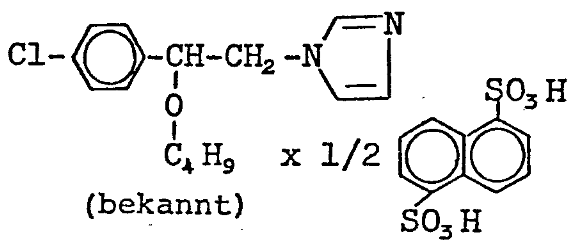 x 1/2 (bekannt) | 48 |
| 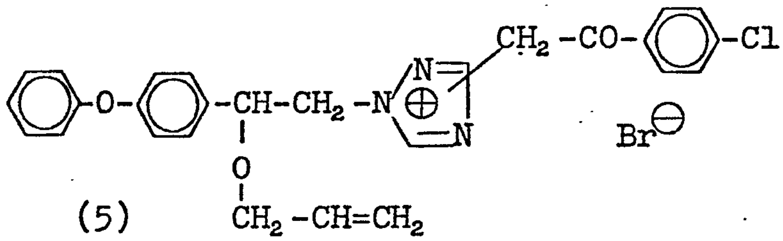 (5) | 5 |
| 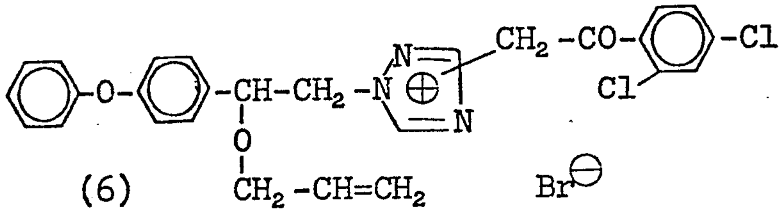 (6) | 4 |
| 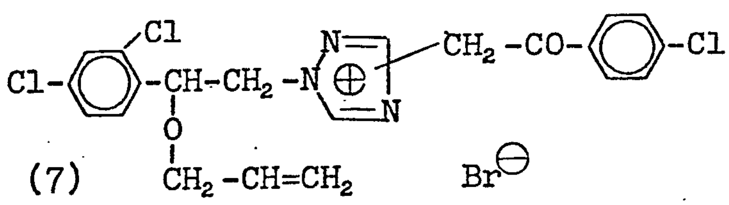 (7) | 1 |
| 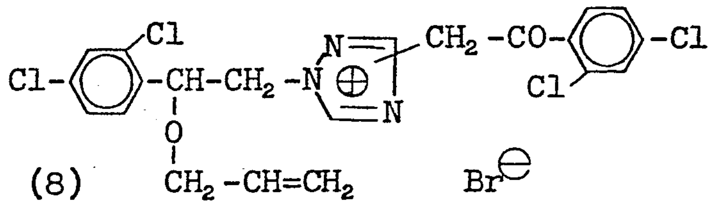 (8) | 1 |

Tabelle A (Fortsetzung)

Podosphaera-Test (Apfel)/Protektiv

| Wirkstoff | Befall in % bei einer Wirkstoffkonzentration von 0,0025% |
|---|---|
| 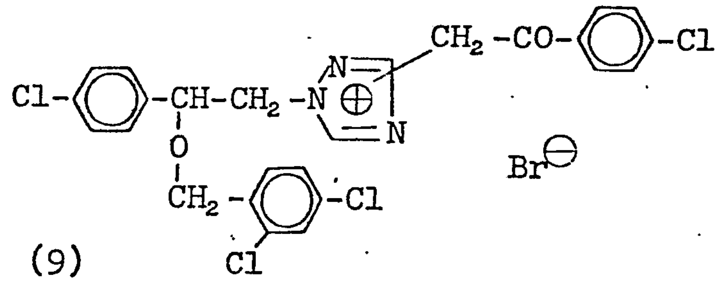 (9) | 41 |
| 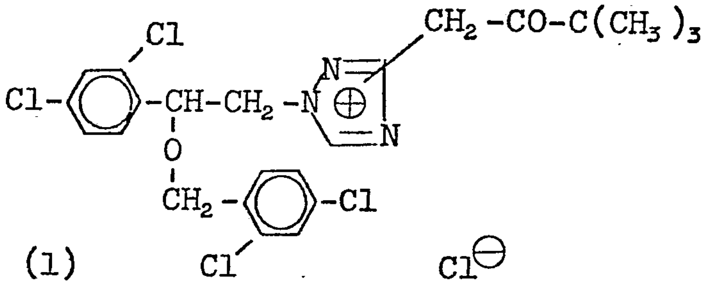 (1) | 0 |
| 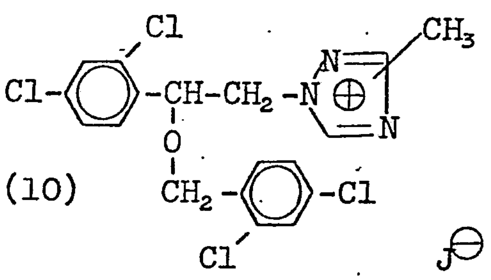 (10) | 31 |

Beispiel B
Fusicladium-Test (Apfel)/Protektiv

| | | |
|---|---|---|
| Lösungsmittel: | 4,7 | Gewichtsteile Aceton |
| Emulgator: | 0,3 | Gewichtsteile Alkyl-arylpolyglykoläther |
| Wasser: | 95,0 | Gewichtsteile |

Man vermischt die für die gewünschte Wirkstoffkonzentration in der Spritzflüssigkeit nötigen Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wässrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse gehen aus der nachfolgenden Tabelle hervor:

Tabelle B

Fusicladium-Test (Apfel)/Protektiv

| Wirkstoff | Befall in % bei einer Wirkstoffkonzentration von |
| --- | --- |
| | 0,0025% |
| 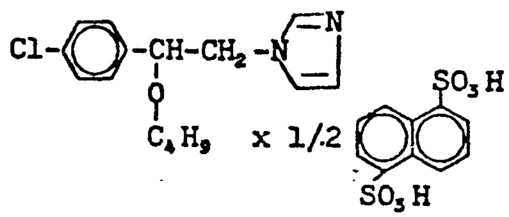 (bekannt) | 51 |
| 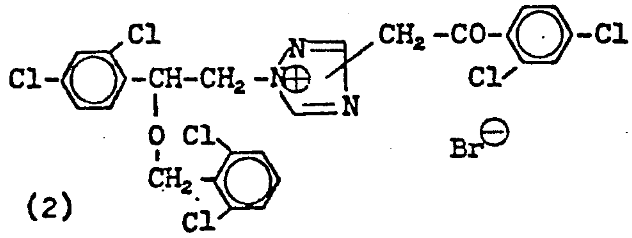 (2) | 1 |
| 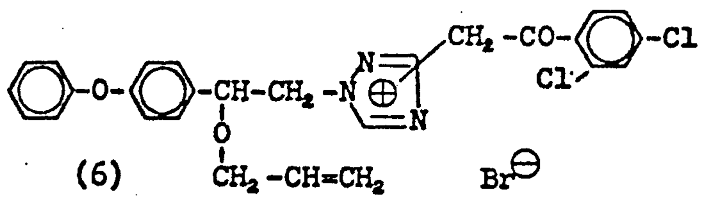 (6) | 39 |
| 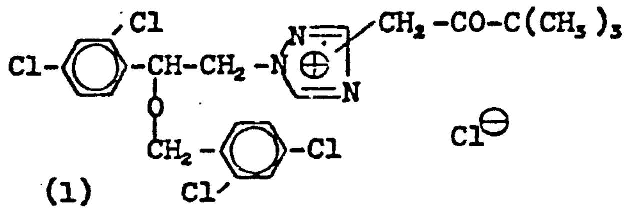 (1) | 7 |

Beispiel C

Sproßbehandlungs-Test/Getreidemehltau/protektiv

(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Alkylaryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0%

keinen Befall und 100% den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrade gehen aus der nachfolgenden Tabelle hervor.

Tabelle C

Sproßbehandlungs-Test/Getreidemehltau/protektiv

| Wirkstoffe | Wirkstoffkonzentration in der Spritzbrühe in Gew.% | Befall in % der unbehandelten Kontrolle |
|---|---|---|
| unbehandelt | — | 100 |
| 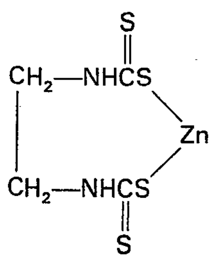 (bekannt) | 0,025 | 100 |
| 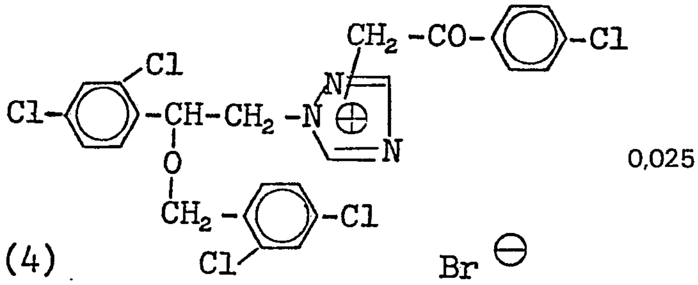 (4) | 0,025 | 25,0 |
| 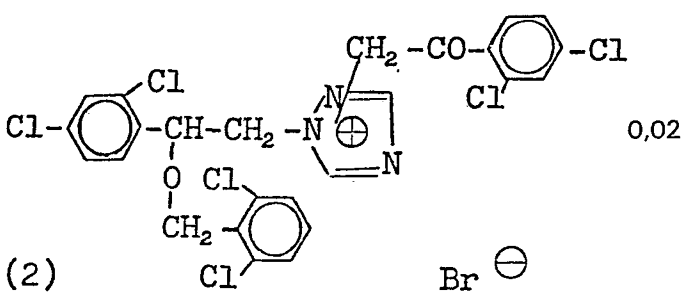 (2) | 0,025 | 16,3 |
| 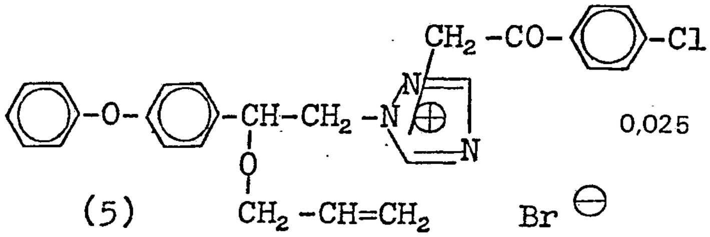 (5) | 0,025 | 42,5 |

Tabelle C (Fortsetzung)

Sproßbehandlungs-Test/Getreidemehltau/protektiv

| Wirkstoffe | Wirkstoffkonzentration in der Spritzbrühe in Gew. % | Befall in % der unbehandelten Kontrolle |
|---|---|---|
| 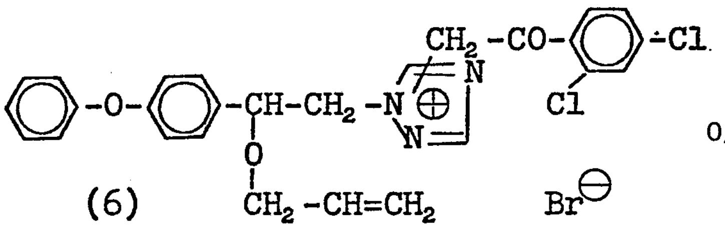 (6) | 0,025 | 0,0 |
| 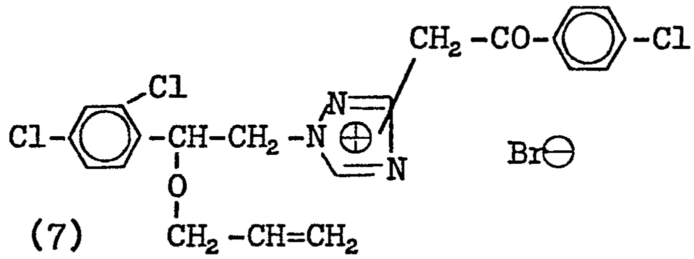 (7) | 0,025 | 0,0 |
| 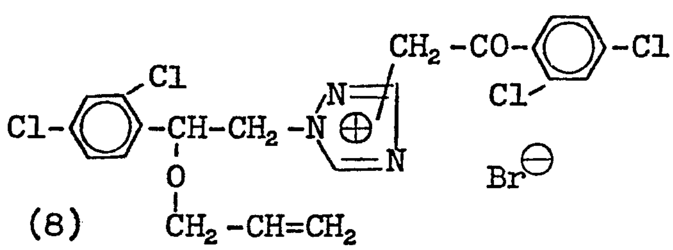 (8) | 0,025 | 0,0 |
| 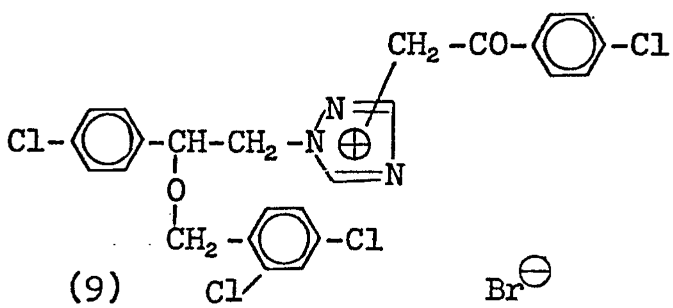 (9) | 0,025 | 12,5 |

Beispiel D

Gerstenmehltau-Test (Erysiphe graminis var. hordei)/systemisch
(pilzliche Getreidesproßkrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe

graminis var. hordei bestäubt und bei 21 bis 22°C und 80 bis 90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall gehen hervor aus der nachfolgenden Tabelle.

Tabelle D

Gerstenmehltau-Test (Erysiphe graminis var.hordei)
systemisch

| Wirkstoffe | Wirkstoff-konzentration im Beizmittel in Gew.% | Beizmittel-aufwandmenge in g/kg Saatgut | Befall in % der unbe-handelten Kontrolle |
|---|---|---|---|
| ungebeizt | — | — | 100 |
| 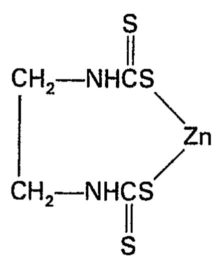 (bekannt) | 25 | 10 | 100 |
| 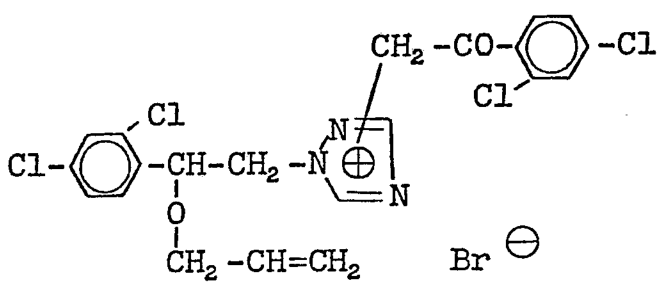 (8) | 25 | 10 | 16,3 |

Beispiel E
Myzelwachstums-Test

Verwendeter Nährboden:

| | |
|---|---|
| 20 | Gewichtsteile Agar-Agar |
| 200 | Gewichtsteile Kartoffeldekokt |
| 5 | Gewichtsteile Malz |
| 15 | Gewichtsteile Dextrose |
| 5 | Gewichtsteile Pepton |
| 2 | Gewichtsteile $Na_2HPO_4$ |
| 0,3 | Gewichtsteile $Ca(NO_3)_2$ |

Verhältnis von Lösungsmittelgemisch zum Nährboden:

| | |
|---|---|
| 2 | Gewichtsteile Lösungsmittelgemisch |
| 100 | Gewichtsteile Agarnährboden |

Zusammensetzung Lösungsmittelgemisch

| | |
|---|---|
| 0,19 | Gewichtsteile DMF oder Aceton |
| 0,01 | Gewichtsteile Emulgator Alkylaryl-polyglykoläther |
| 1,80 | Gewichtsteile Wasser |
| 2 | Gewichtsteile Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach der Wachstumsgeschwindigkeit der Pilze nach 4—10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

| | |
|---|---|
| 1 | kein Pilzwachstum |
| bis 3 | sehr starke Hemmung des Wachstums |
| bis 5 | mittelstarke Hemmung des Wachstums |
| bis 7 | schwache Hemmung des Wachstums |
| 9 | Wachstum gleich der unbehandelten Kontrolle |

Wirkstoffe, Wirkstoffkonzentrationen und Resultate gehen aus der nachfolgenden Tabelle hervor:

Tabelle E

Mycelwachstums-Test

| Wirkstoffe | Wirkstoff-konzen-tration in ppm | Pilze: Colleto-trichum coffe-anum | Verti-cillium albo-atrum | Helmintho-sporium gramineum | Phythoph-thora cactrorum |
|---|---|---|---|---|---|
| 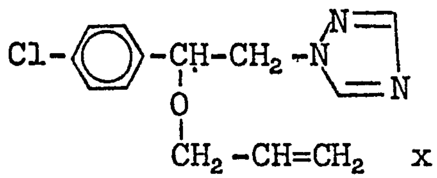 x HCl (bekannt) | 10 | 9 | 5 | 5 | 9 |
| 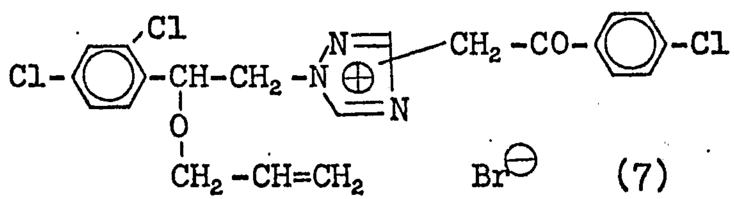 (7) | | 5 | 3 | 3 | 3 |
| 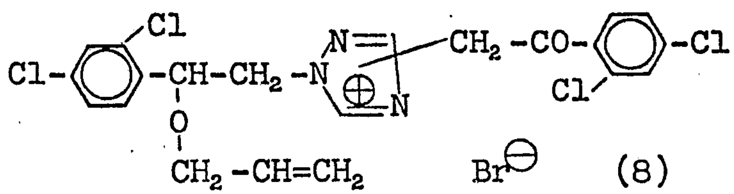 (8) | | 5 | 3 | 1 | 5 |

Herstellungsbeispiele

Beispiel 1

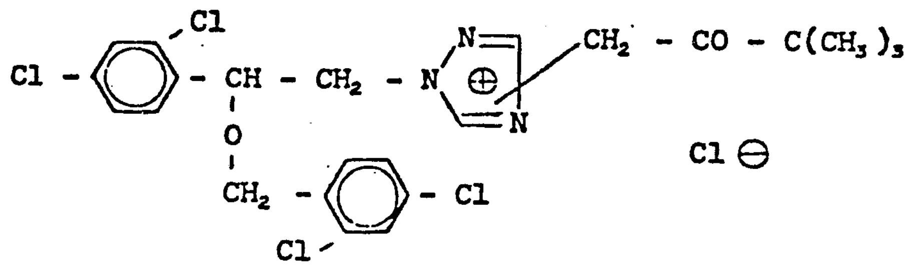

20,7 g (0,05 Mol) [1-(2,4-Dichlorphenyl)-2-(1,2,4-triazol-1-yl)-äthyl]-(2,4-dichlorbenzyl)-äther und 6,7 g (0,05 Mol) Monochlorpinakolin werden in 50 ml Acetonitril 12 Stunden unter Rückfluß erhitzt. Danach läßt man abkühlen, saugt den ausgefallenen Feststoff ab, wäscht mit Aceton nach und trocknet. Man erhält 10,9 g (40% der Theorie) 1-[2-(2,4-Dichlorbenzyloxy)-2-(2,4-dichlorphenyl)-äthyl]-tert.-butylcarbonylmethyl-1,2,4-triazolium-chlorid vom Schmelzpunkt 216—218°C.

Herstellung der Vorprodukte

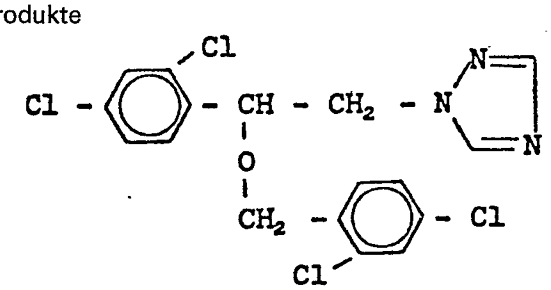

25,8 g (0,1 Mol) 1-Hydroxy-1-(2,4-dichlorphenyl)-2-(1,2,4-triazol-1-yl)-äthan werden in 125 ml Dioxan gelöst und unter Rühren zu einem Gemisch aus 4 g 80%igem Natriumhydrid und 100 ml Dioxan getropft. Danach wird eine Stunde unter Rückfluß erhitzt. Nach dem Abkühlen werden be Raumtemperatur zu dem so erhaltenen Natriumsalz 20 g (0,1 Mol) 2,4-Dichlorbenzylchlorid zugetropft. Anschließend erhitzt man mehrere Stunden unter Rückfluß, läßt abkühlen und engt durch Abdestillieren des Lösungsmittels ein. Der Rückstand wird mit Wasser und Methylenchlorid versetzt, die organische Phase abgetrennt, über Natriumsulfat getrocknet und eingeengt. Der feste Rückstand wird aus Ligroin umkristallisiert. Man erhält 29 g (70% der Theorie) [1-(2,4-Dichlorphenyl)-2-(1,2,4-triazol-1-yl)-äthyl]-(2,4-dichlorbenzyl)-äther vom Schmelzpunkt 84°C.

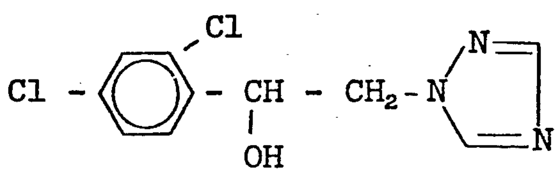

25,6 g (0,1 Mol) $\omega$-(1,2,4-Triazol-1-yl)-2,4-dichloracetophenon werden in 300 ml Methanol gelöst und bei 5 bis 10°C unter Rühren portionsweise mit 4 g (0,1 Mol) Natriumborhydrid versetzt. Anschließend wird eine Stunde bei Raumtemperatur nachgerührt und eine Stunde zum Sieden erhitzt. Nach Abdestillieren des Lösungsmittels wird der Rückstand mit 200 ml Wasser und 40 ml konzentrierter Salzsäure kurzzeitig erhitzt. Nachdem das Reaktionsgemisch mit Natronlauge alkalisch gemacht wurde, kann das feste Reaktionsprodukt abfiltriert werden. Nach dem Umkristallisieren aus Ligroin/Isopropanol erhält man 21,3 g (82% der Theorie) 1-Hydroxy-1-(2,4-dichlorphenyl)-2-(1,2,4-triazol-1-yl)-äthan vom Schmelzpunkt 90°C.

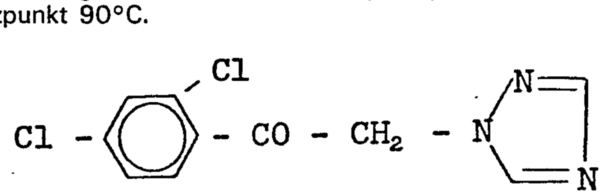

269 g (1 Mol) ω-Brom-2,4-dichloracetophenon werden in 250 ml Acetonitril gelöst. Die Lösung tropft man zu einer unter Rückfluß siedenden Suspension von 69 g (1 Mol) 1,2,4-Triazol und 150 g Kaliumcarbonat in 2 l Acetonitril. Nach 20-stündigem Erhitzen unter Rückfluß wird die erkaltete Suspension filtriert, das Filtrat vom Lösungsmittel befreit und der Rückstand mit.

Essigester aufgenommen, mit Wasser gewaschen, über Natriumsulfat getrocknet und vom Lösungsmittel befreit. Der Essigester-Rückstand kristallisiert beim Versetzen mit Isopropanol aus. Nach dem Umkristallisieren aus Ligroin/Isopropanol erhält man 154 g (60% der Theorie) ω-(1,2,4-Triazol-1-yl)-2,4-dichloracetophenon vom Schmelzpunkt 117°C.

Beispiel 2

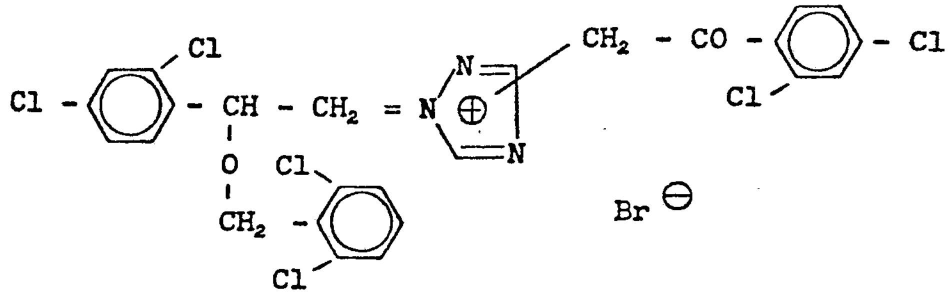

20,7 g (0,05 Mol) [1-(2,4-Dichlorphenyl)-2-(1,2,4-triazol-1-yl)-äthyl]-(2,6-dichlorbenzyl)-äther und 13,4 g (0,05 Mol) 2,4-Dichlorbenzoylmethylbromid werden in 250 ml Chloroform 15 Stunden unter Rückfluß erhitzt. Man läßt abkühlen und engt durch Abdestillieren des Lösungsmittels im Vakuum ein. Der Rückstand wird mit Aceton kurz aufgekocht. Nach dem Abkühlen wird der kristalline Rückstand abgesaugt, mit Aceton gewaschen und getrocknet. Man erhält 5,6 g (16,5% der Theorie) 1-[2-(2,6-Dichlorbenzyloxy)-2-(2,4-dichlorphenyl)-äthyl]-2,4-dichlorbenzoylmethyl-1,2,4-triazolium-bromid vom Schmelzpunkt 202°C.

Beispiel 3

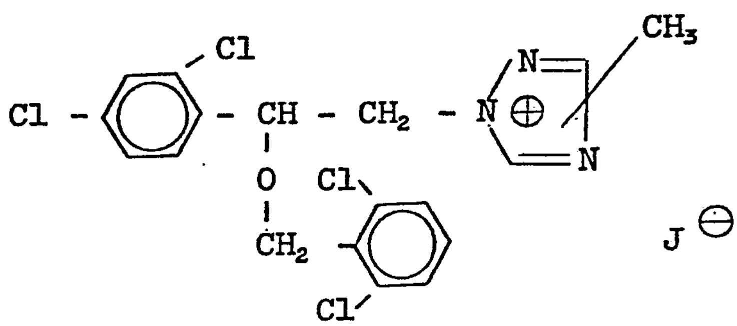

20,7 g (0,05 Mol) [1-(2,4-Dichlorphenyl)-2-(1,2,4-triazol-1-yl)-äthyl]-(2,6-dichlorbenzyl)-äther und 7,1 g (0,05 Mol) Methyljodid werden in 100 ml Acetonitril 12 Stunden unter Rückfluß erhitzt. Danach läßt man abkühlen, saugt den ausgefallenen Feststoff ab, wäscht mit Aceton nach und trocknet. Man erhält 26,7 g (96,7% der Theorie) 1-[2-(2,6-Dichlorbenzyloxy)-2-(2,4-dichlorphenyl)-äthyl]-methyl-1,2,4-triazolium-jodid vom Schmelzpunkt 228—230°C (Zers.).

In analoger Weise werden die folgende Verbindungen der Formel I erhalten:

Tabelle 4

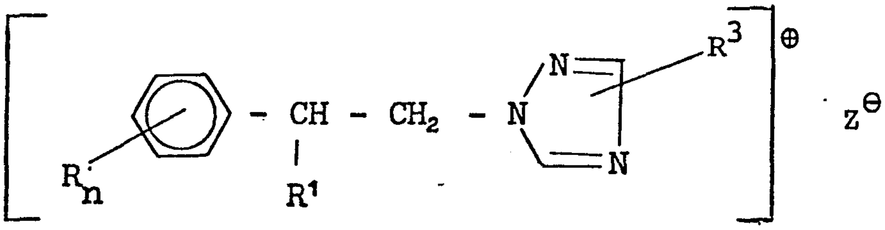

| Bsp. Nr. | $R_n$ | $R^1$ | $R^3$ | Z | Schmelzpunkt (°C) |
|---|---|---|---|---|---|
| 4 | 2,4—Cl | 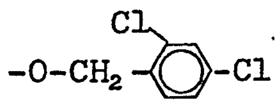 | 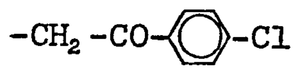 | Br | 228 |
| 5 | 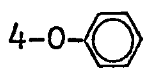 | $—O—CH_2—CH=CH_2$ | 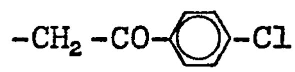 | Br | 206 |
| 6 | 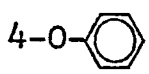 | $—O—CH_2—CH=CH_2$ | 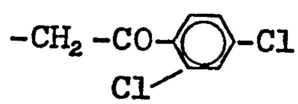 | Br | 172 |
| 7 | 2,4—Cl | $—O—CH_2—CH=CH_2$ | 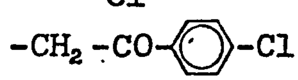 | Br | 161 |
| 8 | 2,4—Cl | $—O—CH_2—CH=CH_2$ | 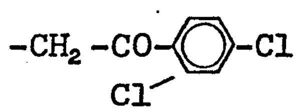 | Br | 183 |
| 9 | 4—Cl | 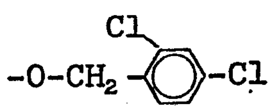 | 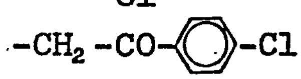 | Br | 233 |
| 10 | 2,4—Cl | 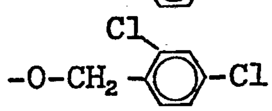 | $CH_3$ | J | 221 |
| 11 | 2,4—Cl | $—O—CO—C(CH_3)_3$ | $CH_3$ | J | 212 |
| 12 | 2,4—Cl | $—O—CO—C(CH_3)_3$ | 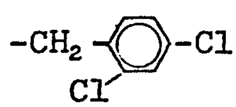 | Cl | 208 |
| 13 | 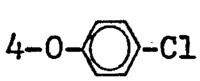 | $—O—CH_2—CH=CH_2$ | 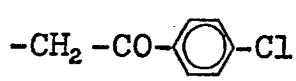 | Br | 150 |
| 14 | 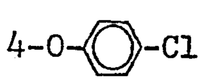 | $—O—CH_2—CH=CH_2$ | $CH_3$ | J | 104 |
| 15 | 2,4—Cl | $—O—CO—CH_3$ | $CH_3$ | J | 171 |
| 16 | 2,4—Cl | $—O—CO—CH_3$ | 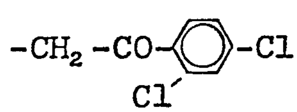 | Br | 174 |

Tabelle 4 (Fortsetzung)

| Bsp. Nr. | $R_n$ | $R^6$ | $R^3$ | Z | Schmelzpunkt (°C) |
|---|---|---|---|---|---|
| 17 | 2,4—Cl | $—O—CH_2—CH=CH_2$ | 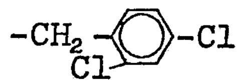 | Cl | 132 |
| 18 | 2,4—Cl | $—O—CH_2—CH=CH_2$ | $—CH_2—CO—C(CH_3)_3$ | Br | 151—153 |
| 19 | 2,4—Cl | $—O—CH_2—CH=CH_2$ | 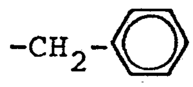 | Br | 108—110 |
| 20 | 2,4—Cl | $—O—CH_2—CH=CH_2$ | $CH_3$ | Br | 55 |

In entsprechender Weise können die folgenden Verbindungen der allgemeinen Formel I hergestellt werden:

| $R_n$ | $R^1$ | $R^3$ | Z |
|---|---|---|---|
| 2,4—Cl | $—S—CH_3$ | 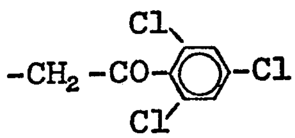 | Br |
| 2,4—Cl | $—S—C_2H_5$ | 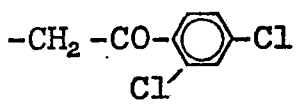 | Br |
| 2,4—Cl | $—S—C_4H_9$ | 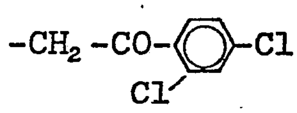 | Br |

**Patentansprüche**

1. 1-(2-Phenyläthyl)-triazolium-Salze der allgemeinen Formel I

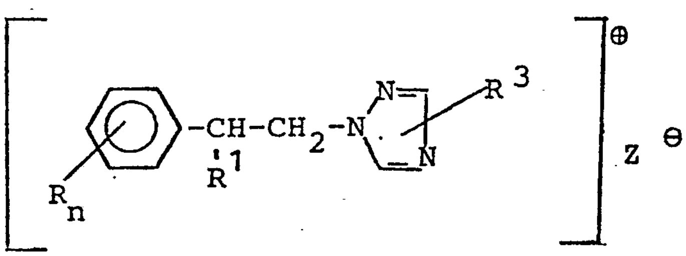

(I)

in welcher

R für Halogen, Alkyl mit 1 bis 4 Kohlenstoffatomen, gegebenenfalls durch Halogen substituiertes Phenyl oder Phenoxy steht,

n für ganze Zahlen von 0 bis 3 steht,

$R^1$ für die Gruppe $—O—R^2$, für Alkylmercapto oder Alkylcarbonyloxy mit jeweils 1 bis 4 Kohlenstoffatomen im Alkylteil steht,

$R^2$ für Alkenyl mit 2 bis 4 Kohlenstoffatomen und für gegebenenfalls durch Halogen substituiertes Benzyl steht,

$R^3$ für Alkyl mit 1 bis 6 Kohlenstoffatomen, für Pinakolyl und für gegebenenfalls durch Halogen substituiertes Benzyl oder Benzoylmethyl steht, und

Z für Halogen steht.

2. Verfahren zur Herstellung von 1-(2-Phenyläthyl)-triazolium-Salzen nach Anspruch 1, dadurch gekennzeichnet, daß man 1-Phenyl-2-triazolyläthyl-Derivate der allgemeinen Formel II

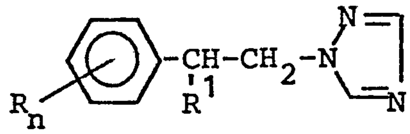

(II)

in welcher

R, $R^1$ und n die in Anspruch 1 angegebene Bedeutung haben,

mit Halogeniden der allgemeinen Formel III

$$R^3—X \qquad (III)$$

in welcher

$R^3$ die in Anspruch 1 angegebene Bedeutung hat,

in Gegenwart eines Verdünnungsmittels in an sich bekannter Weise umsetzt und gegebenenfalls das Halogenid in den so erhaltenen Triazolium-Halogeniden ebenfalls in an sich bekannter Weise gegen ein anderes Halogenid austauscht.

3. Fungizide Mittel, gekennzeichnet durch einen Gehalt an mindestens einem 1-(2-Phenyläthyl)-triazolium-Salz gemäß Anspruch 1.

4. Verwendung von 1-(2-Phenyläthyl)-triazolium-Salzen gemäß Anspruch 1 zur Bekämpfung von Pilzen.

**Revendications**

1. Sels de 1-(2-phényléthyl)-triazolium de formule générale:

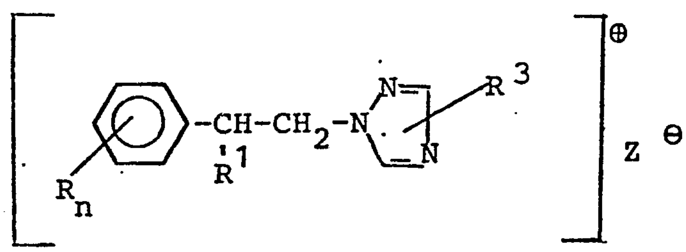

(I)

dans laquelle

R est en halogène, un groupe alkyle ayant 1 à 4 atomes de carbone, phényle ou phénoxy éventuellement substitués par un halogène,

n représente les nombres entiers de 0 à 3,

$R^1$ est le groupe —O—$R^2$, un groupe alkylmercapto ou un groupe alkylcarbonyloxy ayant 1 à 4 atomes de carbone dans chaque partie alkylique,

$R^2$ est un groupe alcényle ayant 2 à 4 atomes de carbone et un groupe benzyle éventuellement substitué par un halogène,

$R^3$ est un groupe alkyle ayant 1 à 6 atomes de carbone, le groupe pinacolyle et un groupe benzyle ou benzoylméthyle éventuellement substitués par un halogène, et

Z est un halogène

2. Procédé de production de sels de 1-(2-phényléthyl)-triazolium suivant la revendication 1, caractérisé en ce qu'on fait réagir d'une manière connue des dérivés 1-phényl-2-triazolyléthyliques de formule générale:

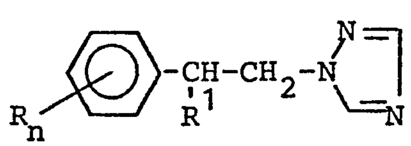

(II)

dans laquelle

R, $R^1$ et n ont la définition indiquée dans la revendication 1, avec des halogénures de formule générale:

$$R^3—X \qquad (III)$$

dans laquelle

$R^3$ a la définition indiquée dans la revendication 1

en présence d'un diluant et, le cas échéant, on échange également d'une manière connue l'halogénure, dans les halogénures de triazolium ainsi obtenus, contre un autre halogénure.

3. Compositions fongicides, caractérisées par une teneur en au moins un sel de 1-(2-phényléthyl)-triazolium suivant la revendication 1.

4. Utilisation de sels de 1-(2-phényléthyl)-triazolium suivant la revendication 1 dans la lutte contre des champignons.

**Claims**

1. 1-(2-Phenylethyl)-triazolium salts of the general formula

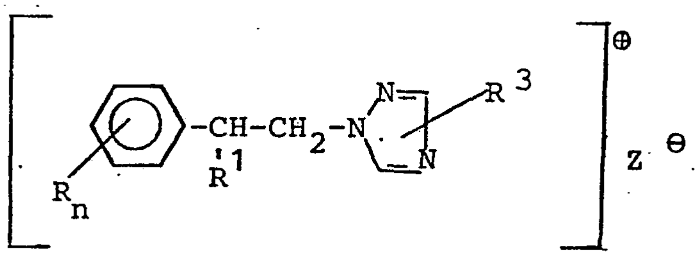

(I)

in which

R represents halogen, alkyl with 1 to 4 carbon atoms and phenyl or phenoxy optionally substituted by halogen,

n represents integers from 0 to 3,

$R^1$ represents the group $—O—R^2$, alkylmercapto or alkylcarbonyloxy with in each case 1 to 4 carbon atoms in the alkyl part,

$R^2$ represents alkenyl with 2 to 4 carbon atoms and benzyl optionally substituted by halogen,

$R^3$ represents alkyl with 1 to 6 carbon atoms, pinacolyl and benzyl or benzoylmethyl optionally substituted by halogen and

Z represents halogen.

2. Process for the preparation of 1-(2-phenylethyl)-triazolium salts according to Claim 1, characterised in that 1-phenyl-2-triazolyl-ethyl derivatives of the general formula

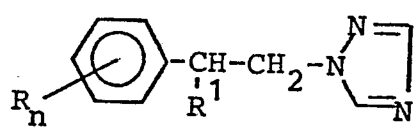

(II)

in which

R, $R^1$ and n have the meaning given in Claim 1 are reacted with halides of the general formula

$$R^3—X \qquad (III)$$

in which

$R^3$ has the meaning given in Claim 1,

in the presence of a diluent in a manner known per se and the halide in the resulting triazolium halides is optionally replaced by another halide also in a manner known per se.

3. Fungicidal agents characterised in that they contain at least one 1-(2-phenylethyl)-triazolium salt according to Claim 1.

4. Use of 1-(2-phenylethyl)-triazolium salts according to Claim 1 for combating fungi.